# EUROPEAN PATENT APPLICATION

(11) **EP 4 480 532 A1**
(43) Date of publication of application: **25.12.2024**
(21) Application number: 23180402.2
(22) Date of filing: 20.06.2023
(51) Int. Cl.: A61N 1/39, H04L 67/12

(54) **IOT DEVICE FOR AN AUTOMATED EXTERNAL DEFIBRILLATOR**

(71) Applicant: Raycap, S.A., 15124 Athens (GR)
(72) Inventor: Giannopoulos, Athanasios, 66100 Drama (GR); Oikonomidis, Christos, 66100 Drama (GR); Rallis, Georgios, 66100 Drama (GR)
(74) Representative: Kouzelis, Dimitrios

(57) **Abstract**

There is provided an Internet of things (IoT) device that is configured to be mounted within an automated external defibrillator (AED), wherein the IoT device comprises a printed circuit board, at least one power source and a communication module. The printed circuit board (20) is configured to be remotely connected with an Internet of Things platform for collecting data from the automated external defibrillator. The Internet of Things device also comprises a global positioning system antenna (30) that is configured to transmit to the Internet of Things platform information about the location of the automated external defibrillator.

## Description

### Technical Field

The present disclosure relates to an Internet of Things device that is configured to be mounted within an automated external defibrillator, and a computer implemented method for tracking an automated external defibrillator.

### Background

Automated External Defibrillators (also mentioned in the current disclosure as AEDs) are commonly designed and used to deliver automated electrical shocks to individuals experiencing sudden cardiac arrest. Cardiac arrest is a life-threatening condition that occurs when the heart suddenly stops beating effectively, leading to a cessation of blood circulation. Rapid response and effective treatment are critical for increasing the chances of survival for individuals experiencing cardiac arrest. Traditional external defibrillators require trained medical professionals to operate them, limiting their availability during the crucial early minutes following cardiac arrest. Automated External Defibrillators were developed to address this issue and provide a solution that can be used by non-medical personnel, including bystanders and first responders. AEDs are portable devices capable of analyzing the heart rhythm of a patient and delivering an electric shock if necessary. The availability and accessibility of AEDs have significantly improved the outcomes of cardiac arrest events by enabling prompt intervention. However, conventional AEDs lack the capability to track and collect comprehensive data regarding their usage and operational parameters. Data collection and analysis play a crucial role in improving medical devices, enabling manufacturers and medical professionals to identify areas for enhancement and make evidence-based decisions. Furthermore, the ability to monitor AED activity in real-time can facilitate remote troubleshooting, maintenance, and timely interventions when necessary. The lack of systematic data gathering affects the ability to analyze and optimize AED performance and reliability. Therefore, existing AEDs still have certain limitations that could be improved.

For example, current AEDs although capable of being used by non-medical personnel, do not provide the option of alerting certified users who may be needed depending on the severity of the medical event. The implementation of a central rescuer database ensures that notifications are exclusively issued to certified personnel, whereas untrained individuals may only alert emergency services or provide support in alternative ways.

To address these challenges, the present disclosure provides an Internet of Things (also mentioned in the current disclosure as loT) device that is mounted within an AED. The loT device allows for the collection of various parameters during the operation of the AED, such as time of deployment, user interactions, and device status. By securely transmitting this data to a central database or cloud-based platform, the loT device enables medical professionals or authorities to gain valuable insights into AED performance. The seamless data collection and remote monitoring capabilities provided by the loT device will enhance the efficiency, reliability, and overall impact of AEDs in saving lives.
During medical emergencies such as cardiac arrests, quick access to an AED and prompt assistance from certified rescuers can significantly improve survival rates. However, locating the nearest available AED and mobilizing the appropriate responders can be challenging, leading to delays in providing life-saving interventions. There is a need for an integrated system that efficiently coordinates AED deployment, alerts nearby responders, and communicates with emergency services to optimize emergency response efforts.

The herein disclosed system is therefore related to the field of emergency response systems, is integrated to Automated External Defibrillators and incorporates real-time AED tracking, automated alert mechanisms, and communication with emergency services to improve the response time and efficiency during emergency situations.

### Summary

The present disclosure relates to an Internet of Things device according to claim 1, an automated external defibrillator according to claim 9, and a computer implemented method for tracking an automated external defibrillator according to claim 10.

According to a first aspect of the disclosure an Internet of Things device is provided, that is configured to be mounted within an automated external defibrillator, wherein the Internet of Things device comprises a printed circuit board, at least one power source and a communication module.

In embodiments, the communication module is a Long-Range module.

In embodiments, the printed circuit board is configured to be remotely connected with an Internet of Things platform for collecting data from the automated external defibrillator.

In embodiments, the Internet of Things device comprises a global positioning system antenna that is configured to transmit to the loT platform information about the location of the AED.

In embodiments, the Internet of Things device comprises a motion detection sensor, in particular an accelerometer sensor.

In embodiments, the Internet of Things device comprises a photosensor that is configured to detect the color of the element for indicating the remaining life of the at least one or more batteries of the automated external defibrillator.

In embodiments, the Internet of Things device comprises at least one environmental sensorthat is connected to the printed circuit board and is configured to collect environmental data of the automated external defibrillator.

In embodiments, the environmental sensor comprises a humidity sensor and a temperature sensor.

According to a second aspect of the disclosure, an automated external defibrillator is provided, comprising one or more batteries, a capacitor for storing energy, a pair of electrode pads for providing an electrical shock to a patient during a cardiac event, a processor for analyzing a patient cardiac rhythm, an element for indicating the remaining life of the at least one or more batteries of the automated external defibrillator, wherein said element comprises an illuminating device that is configured to emit a variable color, depending on the level of the remaining life of the one or more batteries, and the loT device according to the first aspect of the disclosure.

According to a third aspect of the disclosure, a computer implemented method for tracking an automated external defibrillator is provided comprising the steps of:
- Locating, via a software application that is installed in a mobile electronic device, the location of an automated external defibrillator that is in the proximity of the location of a medical event,
- Notifying a user about the operating status of the automated external defibrillator, wherein the operating status comprises either an active status where the automated external defibrillator is ready for use, or a maintenance status where the automated external defibrillator is not ready for use, wherein when the automated external defibrillator is not ready for use, the user is directed to another automated external defibrillator, depending on the distance from the occurrence of the medical event.
- Transmitting a signal, more specifically an emergency signal, to a communication station when the automated external defibrillator is moved from a predefined location, thereby notifying the communication station about the occurrence of a medical event.

In embodiments, the method comprises the steps of
- Registering a plurality of medical responders to a database
- Identifying the location of the registered medical responders wherein
- Transmitting an emergency signal further comprises alerting one or more of the plurality of medical responders through a mobile device, wherein the signal is only transmitted to a first group of medical responders of the plurality of the medical responders, wherein the current position of the first group of medical responders is within a predefined distance from automated external defibrillator.

In embodiments, the method comprises the step of displaying to the first group of medical responders the location of the medical event and the operating status of the automated external defibrillator.

In embodiments, the method comprises the step of applying a predictive maintenance algorithm that is configured to analyze data from the automated external defibrillator and notify the communication station about the maintenance requirements of the automated external defibrillator.

In embodiments, the data that is analyzed by the predictive maintenance algorithm comprises data coming from the battery of the automated external defibrillator, estimating the expiration date of the pair of the electrode pads wherein the method further comprises the step of reporting to the communication station the analyzed data.

According to a fourth aspect of the disclosure, there is provided a computer program configured to execute the computer implemented method for tracking an automated external defibrillator according to the third aspect.

Additional details and features are described in reference to the drawings as follows.

### BRIEF DESCRIPTION OF THE DRAWINGS

Other characteristics will be apparent from the accompanying drawings, which form a part of this disclosure. The drawings are intended to further explain the present disclosure and to enable a person skilled in the art to practice it. However, the drawings are intended as non-limiting examples. Common reference numerals on different figures indicate like or similar features.
**Fig. 1** is a schematic view of automated external defibrillator having the Internet of Things device of the first aspect.
**Fig. 2** is a schematic view of the Internet of Things device.
**Fig. 3** schematically illustrates a computer implemented method for tracking an automated external defibrillator.
**Fig. 4** shows a network diagram of the PCB and of the other components of the Internet of Things device and of the automated external defibrillator.
**Fig.5** shows a protective box having the automated external defibrillator according to aspects of the disclosure.

### DETAILED DESCRIPTION

Embodiments of the loT device will be described in reference to the drawings as follows.

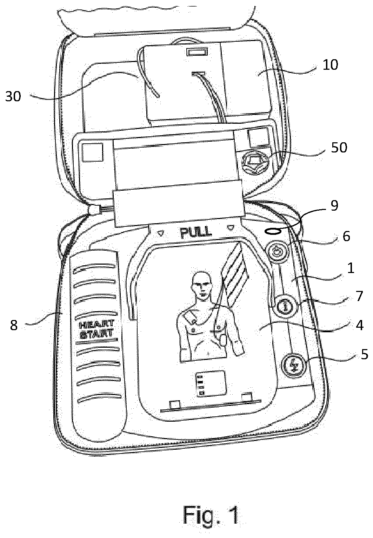

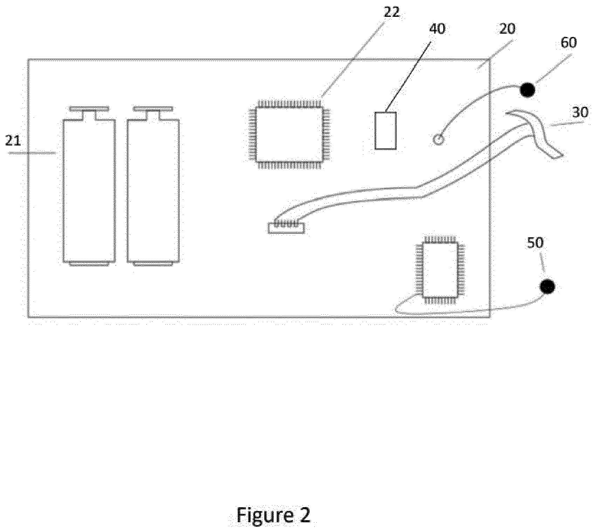

Fig. 1 is a schematic view of an Automated external defibrillator comprising the Internet of things device 10 according to the first aspect. As shown in figure 2. loT device 10 comprises a printed circuit board (also mentioned in the current disclosure as PCB) 20, at least one power source 21 and a communication module 22. Such electronic assembly combines the functionality of providing power and enabling communication in a compact and integrated manner. A PCB 20 may be a flat board made of non-conductive material, such as but not limited to fiberglass or composite epoxy, with conductive tracks etched or printed onto its surface. These tracks form a circuitry that connects various electronic components mounted on the PCB 20. In examples, the power source 21 o includes components such as batteries, voltage regulators and capacitors. It provides the necessary electrical power to operate the components and devices connected to the PCB 20.

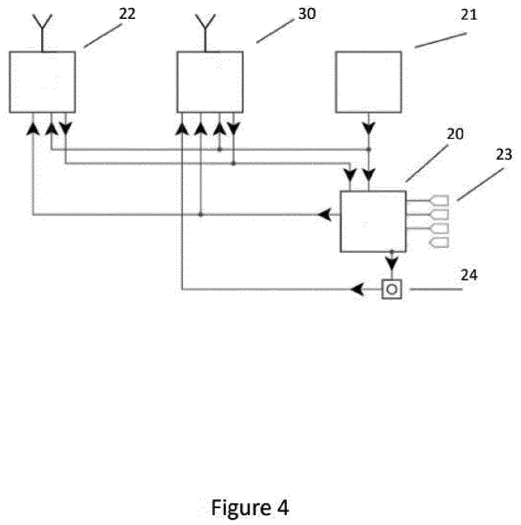

The communication module 22 that is connected to the PCB 20 enables the exchange of data or information or inputs 23 between the PCB 20 and other devices or systems. Module 22 can include components like microcontrollers, transceivers, antennas, etc. It allows the PCB 20 to communicate wirelessly (e.g., using Bluetooth, Wi-Fi,). When combined, the power source 21 and communications module 22 on the PCB 20 provide a self-contained solution for powering and communicating with other devices. In examples, the PCB 20 may also include a microcontroller or a microprocessor, which acts as the brain of the device 10. It controls the communication module 22, manages data collection, performs necessary computations, and ensures the smooth operation of the loT device 10. Figure 4 indicatively provides a schematic diagram of the PCB and of the other components of the loT device.

In examples, the communication module 22 is a Long Range (also mentioned in the current disclosure as LoRa) module. LoRa is a wireless communication technology specifically designed for long-range, low-power applications. The LoRa module 22 can send immediate notifications to designated recipients in emergency situations. When an AED 1 is activated, it can transmit an alert, including location information, to nearby medical professionals or emergency response teams. This rapid communication can significantly reduce response times and increase the chances of successfully handling a cardiac event. LoRa modules are also modules of low power consumption, thereby enabling extended battery life of the loT device. Further, LoRa operates in a frequency range that allows for long-range communication even in challenging environments. This ensures reliable connectivity between the AED 1 and the LoRa network infrastructure, providing robust communication capabilities in various settings, including crowded urban areas or remote locations. In situations where LoRa coverage is not available, a GSM 4G/5G modem is incorporated into the loT device 10. This modem serves as an alternative communication channel, ensuring reliable transmission of data when LoRa connectivity is limited.

In examples, the printed circuit board 20 is configured to be remotely connected with an Internet of Things (also mentioned in the current disclosure as loT) platform for collecting data from the automated external defibrillator. The loT platform serves as the central hub for receiving, processing, and storing data from the AED 1. It acts as an intermediary between the device 10 and authorized users, allowing them to remotely monitor and manage the AED's performance and data. The platform may feature a user-friendly interface that provides real-time updates, analytics, and notifications regarding the AED's status and usage. The connection of device 10 with the loT platform enables real-time data collection from the AED 1, allowing the platform users or the emergency response teams to remotely monitor the device's status and thus decide promptly. The loT platform can also send alerts or notifications in case of any abnormalities or maintenance requirements. The collected data can be utilized for analytics and insights, aiding in the improvement of AED performance, maintenance schedules, and overall effectiveness. It enables proactive maintenance, ensuring that the AED 1 remains in optimal condition for emergencies. To facilitate communication with the database and the loT platform, the loT device 10 may employ the MQTT (Message Queuing Telemetry Transport) protocol. It enables the exchange of JSON messages with the appropriate topic for each defibrillator, ensuring efficient and structured data transmission.

In examples, the loT device 10 comprises a global positioning system antenna 30 that is configured to transmit to the loT platform information about the location of the AED 1. By integrating GPS or other positioning technologies with the LoRa module 22, the AED's location can be accurately determined and transmitted. In cases where multiple AEDs are deployed in a region, this information can assist in directing individuals to the nearest available device during emergencies. When the AED 1 is activated or triggered, the loT device 10 automatically starts collecting GPS data. This information can include the latitude, longitude, altitude, timestamp, providing precise details of the AED's location at the time of the event. The collected GPS data may be transmitted securely to a server or cloud-based platform or communication station, where it can be processed, stored, and made available for further analysis or retrieval. Authorized users can access the platform to view the location history and track the movement of the AED 1. In emergency situations, it enables rapid identification and locating of the AED 1, which can be crucial for saving lives. Emergency responders can quickly navigate to the AED's location, minimizing response time and increasing the chances of successful intervention. The GPS data collected by the loT device 10 also facilitates post-event analysis and reporting. It allows healthcare providers to analyze response patterns, identify areas of improvement, and optimize the placement of AEDs for better coverage in public spaces or facilities. Additionally, the loT device 10 with GPS integration can enhance AED 1 management and maintenance. By tracking the location of each AED 1, organizations can monitor their distribution, perform routine checks, and ensure that AEDs are always readily available in optimal locations.

In examples, the loT device 10 comprises a motion detection sensor 40, in particular an accelerometer sensor 40. Sensor 40 enables device 10 to monitor the AED's physical movement. In case the accelerometer detects motion, the loT device 10 responds by sending measurements, for example, every 10 seconds of the new GPS location, providing real-time tracking until the accelerometer stabilizes. In examples, AED 1 comprises an element 9 for indicating the remaining life of the at least one or more batteries of the AED 1, wherein said element comprises an illuminating device that is configured to emit a variable color, depending on the level of the remaining life of the one or more batteries, wherein the loT device 10 comprises a photosensor 50 that is configured to detect the color of the element 9 for indicating the remaining life of the at least one or more batteries of the AED 1. In that way, AED's battery level can be monitored accurately.

In examples and as indicatively shown in figure 2, the loT device 10 comprises at least one environmental sensor 60 that is connected to the printed circuit board 20 and is configured to collect environmental data of the AED 1. Such environmental sensors may comprise a humidity sensor and a temperature sensor for measuring temperature and humidity. These sensors provide important environmental data that can be used for analysis, ensuring that the AED 1 is stored in appropriate conditions and remains operational when needed.

To conclude, the measurements that the loT device 10 sends to the platform may include latitude/longitude coordinates for location tracking, temperature and humidity data from the sensors, board battery level, X, Y, Z measurements, accelerations, and vibrations captured by the accelerometer. These measurements provide comprehensive insights into the AED's operational status, environmental conditions, and physical movement.

According to the second aspect of the present disclosure, an automated external defibrillator 1 is provided that comprises:
- one or more batteries 2,
- a capacitor for storing energy 3,
- a pair of electrode pads 4 for providing an electrical shock to a patient during a cardiac event
- a processor for analyzing a patient cardiac rhythm and
- and the loT device 10 according to the first aspect.

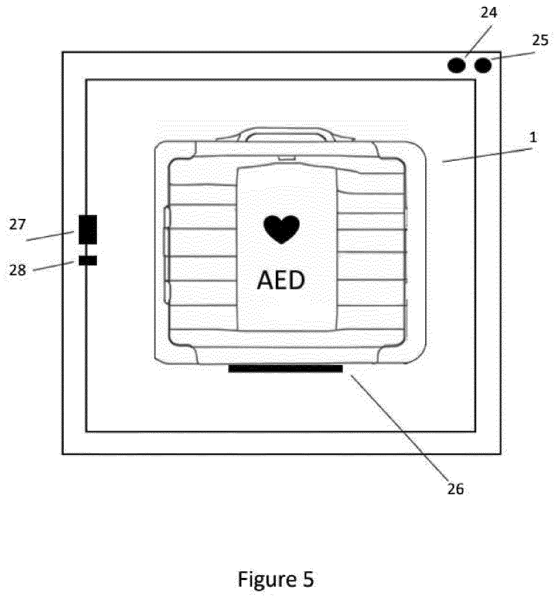

As discussed, automated External Defibrillators (AEDs) are medical devices that are designed to save lives in cases of sudden cardiac arrest. During such a critical event, AEDs play a crucial role in increasing the chances of survival. AEDs are compact, portable devices that deliver an electric shock to the heart to restore its normal rhythm. They are designed to be user-friendly, enabling even non-medical personnel to provide immediate assistance until professional medical help arrives. As can be seen in figure 1, these devices typically comprise of a control unit, electrode pads 4, and an internal computer system having a processor that analyzes the heart rhythm and determines if a shock is necessary. They may further comprise an electrocute button 5 that is used to initiate the delivery of an electrical shock to the patient, a power on/off actuator 6 that enables the user to activate or deactivate the AED, and an information button 7 that allows a user to access and view status information that is related to the AED, including but not limited to battery status, electrode pad expiration dates etc. AEDs may be mounted within a protective case 8 and may be strategically placed in public areas, including airports, schools, sports facilities, shopping centers, and workplaces. Their widespread availability is vital because the first few minutes following cardiac arrest are critical for successful resuscitation. Studies have shown that prompt defibrillation with an AED 1, within the first few minutes, can significantly improve the chances of survival. The use of AEDs is not limited to medical professionals. With appropriate training and basic knowledge of cardiopulmonary resuscitation (CPR), bystanders and individuals with minimal medical training may effectively operate these devices. In addition, by utilizing the herein disclosed loT device, security of the AED in a public space is enhanced since setup addons may be implemented. Figure 5 shows a protective box where there is located an automatic external defibrillator that has mounted the loT device of the current disclosure. The usage of such loT device allows the box to be equipped for example with a camera 24, a smart door lock 25 that allows only specific users to unlock the AED 1 and a door alarm 27 that is actuated in case of malicious treatment of the AED, a weight sensor 26 and a motion sensor 28. The camera 24 captures visual data and enables real-time video monitoring of the AED and its surroundings. This visual monitoring enhances the overall security and surveillance capabilities of the AED system. A smart door lock 25 is installed on the protective box to deactivate the emergency response mechanisms. This smart lock 25 may operate using authentication mechanisms or secure digital keys for authorized users, such as maintenance personnel to maintain the AED or the loT device without triggering the emergency system. The protective box may also include a door alarm 27 that is triggered when the door opens. The door alarm may emit an audible alert or send notifications to authorized individuals. Weight sensor 26 may be incorporated into the protective box to detect the presence or removal of the AED 1. Weight sensor 26 can monitor the weight of the AED 1 within the box, alerting authorized individuals if the AED is removed without proper authorization. This weight sensor 26 acts as an additional layer of security and prevents unauthorized handling of the AED.

The herein disclosed AED 1 is equipped with one or more batteries 2, ensuring sufficient power to operate the device for an extended period. The batteries 2 are designed to provide optimal performance and longevity, enabling reliable functionality during critical situations. The AED 1 includes a capacitor 3 that efficiently stores electrical energy required for defibrillation. This capacitor 3 is engineered to deliver rapid and controlled discharges, ensuring the effective transmission of therapeutic shocks to the patient's heart. Further, the AED 1 comprises a pair of electrode pads 4 that are configured to be placed on a patient's chest. These pads are designed to deliver the electrical shock necessary to restore the heart's normal rhythm during a cardiac event. Moreover, AED 1 incorporates a processor capable of analyzing the patient's cardiac rhythm with accuracy and speed. The processor detects and classifies abnormal heart rhythms, providing critical information to guide treatment decisions. The processor's robust capabilities improve the device's diagnostic capabilities and overall effectiveness in delivering appropriate therapeutic interventions. By combining these essential components with the loT device 10 as previously described, the AED 1 system aims to revolutionize the field of cardiac emergency response. The integration of loT technology enhances the device's capabilities, allowing for remote monitoring, data collection, and improved overall performance. This AED 1 design holds significant potential in improving the survival rates of individuals experiencing sudden cardiac arrest.

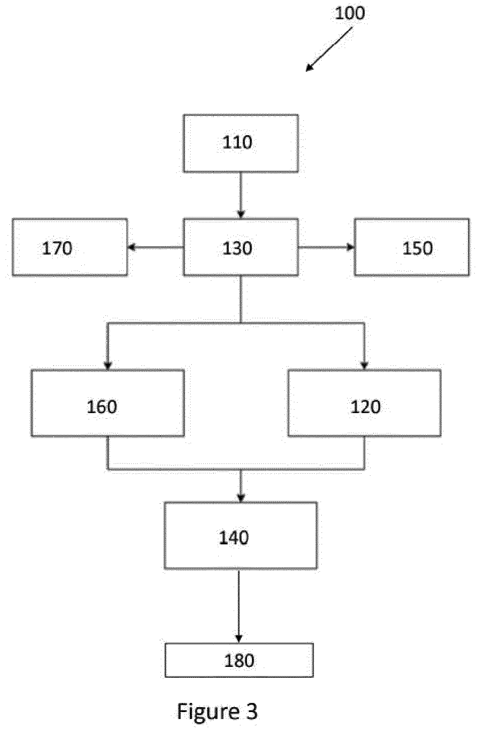

Figures 3 indicatively shows a third aspect of the disclosure, which is a computer implemented method 100 for tracking an automated external defibrillator that comprises the steps of:
- Locating 110, via a software application that is installed in a mobile electronic device, the location of an automated external defibrillator 1 that is in the proximity of the location of a medical event,
- Notifying 120 a user about the operating status of the AED 1, wherein the operating status comprises either an active status where the AED 1 is ready for use, or a maintenance status where the AED 1 is not ready for use, wherein when the AED 1 is not ready for use, the user is directed to another AED 1, depending on the distance from the occurrence of the medical event.
- Transmitting 130 a signal, more specifically an emergency signal, to a communication station when the automated external defibrillator is moved from a predefined location, thereby notifying the communication station about the occurrence of a medical event.

In detail, the method uses a software application installed, for example on a mobile electronic device to determine the location of an AED 1 near the site of a medical event. This could be achieved through various means, such as GPS. Once the AED's location is identified, the method provides information about the operating status of the AED 1. The operating status can be categorized into two states: active and maintenance. If the AED 1 is in an active status, it means the device is ready for use. However, if it is in a maintenance status, it indicates that the AED 1 is not currently available for use, possibly due to maintenance or other reasons. In such cases, the user is directed to another nearby AED 1, considering the distance from the medical event. Further, the method includes transmitting an emergency signal to a communication station when the AED 1 is moved from a predefined location. This signal notifies the communication station about the occurrence of a medical event. The purpose of this step is to alert relevant authorities or emergency responders about the situation so that they can provide appropriate assistance.

In examples, the method may further comprise the steps of
- Registering 150 a plurality of medical responders to a database
- Identifying 160 the location of the registered medical responders wherein
- Transmitting an emergency signal further comprises alerting 140 one or more of the plurality of medical responders through a mobile device, wherein the signal is only transmitted to a first group of medical responders of the plurality of the medical responders, wherein the current position of the first group of medical responders is within a predefined distance from automated external defibrillator.

The presence of a central rescuer database assists in ensuring that only certified individuals are involved in emergency incidents. This database provides an added layer of safety and prevents untrained personnel from attempting rescues, which could potentially be harmful to those in need of assistance. The central rescuer database plays an important role in the system by storing information about certified individuals who are qualified to provide first aid and respond to medical emergencies. When an emergency occurs and an automated external defibrillator is deployed, the system selectively notifies and alerts the appropriate certified rescuers from the database. This ensures that the notifications are exclusively sent to trained individuals who possess the necessary skills to handle the situation effectively. By integrating the central rescuer database, the system distinguishes between certified rescuers and untrained users of the mobile app. Untrained individuals may still be able to use the app to alert emergency services or provide support in other ways, but they are not included in the direct notification process. This safeguard helps prevent unqualified individuals from causing harm to those in need. Including a central rescuer database strengthens the overall safety and effectiveness of the system, ensuring that only certified responders are involved in emergency incidents. The database may include various information for each rescuer, for example, name, address, phone number, certification issue and expiration date, date of registration, number of incidents involved and GPS signal.

After the emergency signal is transmitted 130, it alerts 140 one or more of the registered medical responders through their mobile devices. However, the signal is only transmitted to a first group of medical responders from the plurality of medical responders. The criteria for the first group of medical responders are based on their current position in relation to the predefined distance from the AED. Only those medical responders whose current position falls within this predefined distance will receive the alert on their mobile devices. This approach ensures that the closest and most geographically suitable medical responders are promptly notified of the emergency. By selectively alerting the first group of medical responders within the predefined distance, the method optimizes the response time and increases the likelihood of a rapid and effective intervention. These responders, being near the AED 1, can provide timely assistance in utilizing the device and administering first aid.

Once the first group of medical responders within the predefined distance from the automated external defibrillator has been alerted, the system proceeds to display 170 relevant information to these responders. The system displays 170 the location of the medical event on the responders' mobile devices or other suitable interfaces, for example with the usage of an interactive map. This allows the responders to quickly identify where the emergency is taking place. In examples, the system provides information about the operating status of the AED 1 associated with the medical event. This status indicates whether the AED 1 is currently in an active state, ready for use, or in a maintenance state, indicating that it is not available for immediate use. By presenting the location of the medical event and the operating status of the AED 1 to the first group of medical responders, the system ensures that they have relevant and critical information to effectively respond to the emergency. This allows the responders to navigate to the location and make informed decisions based on the availability and readiness of the AED 1.

In examples, the mobile application utilizes the user's location to identify the nearest AEDs in their current city. These AEDs may be displayed on an interactive map along with information about their current status and maintenance plan. With a simple button press, the app provides directions to the nearest operating AED 1, helping users quickly find a device in case of an emergency. In that war, users can access detailed information about each AED 1, including its exact location and for example any relevant instructions for use. This ensures that users have comprehensive knowledge about the AEDs in their vicinity.

The loT platform offers a second login profile specifically for certified rescuers. Rescuers can indicate their availability to provide first aid during certain times and within a specific distance range of an emergency event. Using the rescuer's current location, the platform determines if they are within the specified range of an emergency event. Rescuers are alerted if they are in proximity, enabling them to respond swiftly. Rescuers can use the mobile app to navigate to the nearest operating AED 1 and provide first aid. This feature streamlines the process of connecting trained rescuers with AEDs, improving emergency response times and outcomes. The loT platform facilitates connections between users and certified rescuers in their area, fostering a community of trained individuals ready to provide first aid and save lives in cardiac emergencies. By enabling users to connect with certified rescuers, the loT platform enhances the accessibility of life-saving care during emergencies, increasing the chances of positive outcomes for those in need.

In examples, the method comprises the step of applying 180 a predictive maintenance algorithm that is configured to analyze data from the AED 1 and notify the communication station about the maintenance requirements of the AED 1. In examples, the data that is analyzed by the predictive maintenance algorithm comprises data coming from the battery of the AED 1, estimating the expiration date of the pair of the electrode pads wherein the method further comprises the step of reporting to the communication station the analyzed data.

This algorithm is designed to identify potential maintenance requirements or issues with the AED 1 based on the data patterns and performance metrics. The algorithm assesses various parameters and indicators, such as battery status, electrode pad expiration, calibration status, and other relevant diagnostic information from the AED 1. By analyzing this data, the algorithm can predict when maintenance or servicing may be required for the AED 1. The algorithm examines the battery status to determine its remaining capacity or estimated lifespan. This helps in predicting when the battery might require replacement or servicing to ensure that the AED 1 remains operational. Additionally, the algorithm estimates the expiration date of the electrode pads, which are an essential component of the AED 1. Electrode pads have a limited life, and it is crucial to monitor their expiration dates to ensure that they are replaced before they become ineffective. When the algorithm detects a potential maintenance requirement or an issue that needs attention, it triggers a notification to the communication station. After analyzing the data, the method includes the step of reporting the analyzed data to the communication station. This reporting mechanism ensures that the communication station is informed about the maintenance requirements of the AED 1, for example related to the battery and the expiration date of the electrode pads. This notification alerts the station about the specific maintenance requirements of the AED 1, allowing for timely actions to be taken. By utilizing a predictive maintenance algorithm, the method enhances the proactive monitoring and management of the AED condition. This helps ensure that the AED 1 remains in optimal working order and is always ready for use in emergency situations. The algorithm-driven notifications to the communication station enable appropriate maintenance activities to be scheduled, performed, or facilitated, contributing to the overall reliability and effectiveness of the AED system.

In examples, a hypothetical scenario may include the following situation: A cardiac event occurs, a citizen observes it and calls an emergency phone number for help. The citizen uses his mobile phone and the respective app to locate the closest AED 1 and receives directions to its location. When the citizen picks up the AED 1, an emergency alert is generated and transmitted to a communication station. Certified rescuers and those who are near the AED 1, for example at a distance that is less than 1km, receive the emergency alert and are notified. A certified rescuer (3) starts to approach the AED 1 that is picked by the citizen and consequently is in motion. Meanwhile, the AEDs that are under maintenance status are not shown to the mobile device of the certified rescuer. Afterwards, the rescuer determines that AED 1 is closer to the incident location and proceeds to assist there. It has to be noted that emergency services at a communication station are simultaneously notified about the incident. After the rescue of the patient at risk, the AED 1 is placed back in its initial position, and maintenance personnel may arrive to check the AED 1 that was previously used. The described scenario demonstrates how the system coordinates the response to an emergency incident involving an AED 1. It leverages the mobile app, AED 1 movement detection, and notifications to ensure that certified rescuers are alerted and directed to the incident location, optimizing the response time, and potentially saving lives.

In addition to the above-described scenario, Figure 6 provides an indicative flowchart of the various actions starting from the identification of a cardiac event up to the provision of assistance by a certified rescuer. A random citizen identifies a cardiac event or witnesses a person collapsing and suspects a cardiac arrest. The random citizen picks up an Automated External Defibrillator and activates it. The AED sends an emergency signal to the communication station. The communication station receives the emergency signal from the AED and initiates the emergency response process. The communication station accesses the database of registered rescuers. The rescuers who are close to the location of the incident are notified via their mobile devices. Such notification includes information about the cardiac event and the precise location. Rescuers use GPS navigation on their mobile devices to get directions to the incident location. They may have access to an interactive map on their mobile devices. The map displays the precise point where the AED is located, helping the rescuer locate it quickly. Once the rescuer aids the patient, the AED is placed back in its initial location. This ensures that the AED is readily available for future use if needed. Afterwards, the relevant maintenance teams are notified about the AED's usage. They can reach the AED's location to proceed with proper maintenance activities, such as checking battery life, replacing pads, or conducting any necessary repairs.

The herein disclosed loT device 10 and method for tracking an AED allows the certified users that are registered to specific database to install a mobile app on their smartphones, communicate with the AED communication station, and receive information about the closest AEDs and their status. They are consequently alerted in case of an emergency and provided with the exact location, thus being able to run to the emergency location to aid. Similar applies also for the uncertified users who can also install the application, for example in their smartphones, and receive information about the closest AEDs and their status. The communication station acts as the central management, monitoring, and response system for the AEDs. Such communication station controls the status of the AEDs (active, inactive, under maintenance), receives data from the AEDs and processes it. It further provides interfaces for emergency services, maintenance personnel, and other stakeholders to interact with the system as well as it facilitates the exchange of information between certified users, emergency services, and AEDs. Moreover, through the loT platform, the users that are responsible for the maintenance of the AED's can access the server through their own interface, monitors and manage the AEDs' maintenance requirements, thereby ensuring that every AED remains active with minimal downtime. This comprehensive approach aims to provide efficient emergency response, real-time information, and proactive maintenance management to ensure the availability and effectiveness of AEDs in saving lives.

According to a fourth aspect of the disclosure a computer program is provided that is configured to execute the computer implemented method for tracking an automated external defibrillator according to third aspect.

## Claims

1. An Internet of Things device (10) configured to be mounted within an automated external defibrillator (1), wherein the Internet of Things device (10) comprises a printed circuit board (20), at least one power source (21) and a communication module (22).

2. The Internet of Things device (10) according to claim 1 wherein the communication module (22) is a Long-Range module.

3. The Internet of Things device (10) according to claims 1-2, wherein the printed circuit board (20) is configured to be remotely connected with an Internet of Things platform for collecting data from the automated external defibrillator (1).

4. The Internet of Things device (10) according to claim 2, comprising a global positioning system antenna (30) that is configured to transmit to the Internet of Things platform information about the location of the automated external defibrillator.

5. The Internet of Things device (10) according to any of the preceding claims comprising a motion detection sensor, in particular an accelerometer sensor (40).

6. The Internet of Things device (10) of any of the preceding claims, comprising a photosensor (50) that is configured to detect a color of an element for indicating the remaining life of an automated external defibrillator.

7. The Internet of Things device (10) of any of the preceding claims comprising at least one environmental sensor (60) that is connected to the printed circuit board (20) and is configured to collect environmental data of an automated external defibrillator (1).

8. The Internet of Things device (10) of claim 7 wherein the environmental sensor (60) comprises a humidity sensor and a temperature sensor.

9. An automated external defibrillator (1) comprising:
- one or more batteries (2),
- a capacitor (3),
- a pair of electrode pads (4) for providing an electrical shock to a patient during a cardiac event
- a processor for analyzing a patient cardiac rhythm
- an element (9) for indicating the remaining life of the at least one or more batteries of the automated external defibrillator (1), wherein said element comprises an illuminating device that is configured to emit a variable color, depending on the level of the remaining life of the one or more batteries,
- and the Internet of Things device (10) according to claims 1-8.

10. A computer implemented method (100) for tracking an automated external defibrillator according to claim 9 comprising:
- Locating (110), via a software application that is installed in a mobile electronic device, the location of an automated external defibrillator that is in the proximity of the location of a medical event,
- Notifying (120) a user about the operating status of the automated external defibrillator, wherein the operating status comprises either an active status where the automated external defibrillator is ready for use, or a maintenance status where the automated external defibrillator is not ready for use, wherein when the automated external defibrillator is not ready for use, the user is directed to another automated external defibrillator, depending on the distance from the occurrence of the medical event.
- Transmitting (130) a signal, more specifically an emergency signal, to a communication station when the automated external defibrillator is moved from a predefined location, thereby notifying the communication station about the occurrence of a medical event.

11. The computer implemented method of claim 10 comprising the steps of
- Registering (150) a plurality of medical responders to a database
- Identifying (160) the location of the registered medical responders wherein
- Transmitting an emergency signal further comprises alerting (140) one or more of the plurality of medical responders through a mobile device, wherein the signal is only transmitted to a first group of medical responders of the plurality of the medical responders, wherein the current position of the first group of medical responders is within a predefined distance from automated external defibrillator.

12. The computer implemented method according to claim 10 comprising displaying (170) to the first group of medical responders the location of the medical event and the operating status of the automated external defibrillator.

13. The computer implemented method according to claims 10-12 comprising applying (180) a predictive maintenance algorithm that is configured to analyze data from the automated external defibrillator and notify the communication station about the maintenance requirements of the automated external defibrillator.

14. The computer implemented method of claim 13 wherein the data that is analyzed by the predictive maintenance algorithm comprises data coming from the battery of the automated external defibrillator (1), thereby estimating the expiration date of the pair of the electrode pads wherein the method further comprises the step of reporting to the communication station the analyzed data.

15. A computer program configured to execute the computer implemented method (100) for tracking an automated external defibrillator according to claims 10-14.
